(19) Europäisches Patentamt

European Patent Office

Office européen des brevets

(11) Publication number: **0 406 542 A1**

## (12) EUROPEAN PATENT APPLICATION

(21) Application number: **90108907.8**

(22) Date of filing: **11.05.90**

(51) Int. Cl.⁵: **C07K 7/08, G01N 33/574**

(30) Priority: **11.05.89 US 350442**

(43) Date of publication of application:
**09.01.91 Bulletin 91/02**

(84) Designated Contracting States:
**BE FR IT**

(71) Applicant: **BIOCHECK, INC.**
**c/o Broventure Co., Inc., 16 W.Madison Street**
**Baltimore, Maryland 21201(US)**

(72) Inventor: **Aurelian, Laure (n.m.i.)**
**3404 Bancroft Road**
**Baltimore, Maryland 21215(US)**
Inventor: **Grillo, Santo L.**
**2615 North Rolling Road**
**Baltimore, Maryland 21207(US)**

(74) Representative: **Liesegang, Roland, Dr.-Ing. et al**
**FORRESTER & BOEHMERT**
**Widenmayerstrasse 4e 4**
**D-8000 München 22(DE)**

(54) A method for detecting precancerous and cancerous cervical intraepithelium.

(57) This invention relates generally to a synthetic peptide, called LA-1, and to related peptides. The invention also relates to antibodies to such peptides and to labeled forms of these antibodies. Labels include cytotoxic agents and agents capable of providing a detectable signal. The invention includes the manufacture of such peptides and antibodies and their use as analytic and diagnostic tools. The invention has diagnostic prognostic value for identifying the carcinoma in situ (CIN) patients at high risk of developing invasive cervical cancer. The antibodies specifically stain typical cervical epithelium from patients with CIN I-III and the invasive cancers. The antibodies are highly specific and rarely react with normal cervical epithelium or other squamous cancers.

# A METHOD FOR DETECTING PRECANCEROUS AND CANCEROUS CERVICAL INTRAEPITHELIUM

## SUMMARY OF THE INVENTION

This invention relates generally to peptides and antibodies, and more specifically to a material called LA-1 which is composed of amino acids and has antigenic activity associated with human intraepithetial neoplasia.

The system has diagnostic-prognostic potential for identifying dysplasia and carcinoma in situ (CIN) patients who are at high risk of developing invasive cervical cancer.

The system includes a synthetic peptide, called LA-1, or a related peptide and an antibody to the peptide. LA-1 corresponds to 13 residues at the amino-terminus of the herpes simplex virus-2 (HSV-2) protein, called ICP-10, which is encoded by the transforming viral DNA sequences.

Antibody to LA-1 specifically stains atypical cervical epithelium from patients with CIN I-III and the invasive cancers. The antibody rarely reacts with normal cervical epithelium or other squamous cancers.

Immunologic markers than can identify the CIN lesions destined to progress to invasive cancer could greatly improve the precision and validity of clinical diagnosis and patient management. The invention describes a set of such markers.

## DISCUSSION OF PRIOR ART PATENTS

A brief discussion of prior art patents is set forth below relating to this subject matter.

U. S. Patent No. 4,132,769 relates to a human cancer associated protein, to processes for its isolation and characterization, to antibodies with specificity against the protein and to diagnosis of and therapy for human cancer using antisera or antibodies to the protein.

U. S. Patent Nos. 4,160,018 and 4,160,019 relate to a process for the isolation of cancer associated polypeptide antigen with a cancer diagnosis method being based on determining the presence of the cancer associated polypeptide antigen.

U. S. Patent No. 4,448,890 relates to specific nucleolar antigens from human malignant tumor specimens, their isolation, extraction, purification, antibody and antisera production, and fuse of the antigens and antibodies in diagnostic procedures.

U. S. Patent No. 4,525,352 relates to an antibody for a human leukemia virus, related peptide and the production of the peptide and antibody.

U. S. Patent No. 4,572,800 relates to a human leukemia virus-related peptide, a proces for its preparation, antibodies to the peptide and a process for production of the antibodies.

U. S. Patent No. 4,572,827 relates to a panel of monoclonal antibodies produced from human gastrointestinal tumors as immunogens useful in the diagnosis of colon cancer.

U. S. Patent No. 4,585,740 relates to monoclonal antibody with specificity to human small cell carcinoma.

U. S. Patent No. 4,693,966 relates to monoclonal antibodies which specifically bind to antigens on surfaces of renal, lung and breast cancer cells, and to intracellular cytoskeletal, nuclear and cytoplasmic reticular antigens.

U. S. Patent No. 4,713,352 relates to monoclonal antibodies and their antigenic specificities in identifying, characterizing and determining prognoses for human renal cancers.

U. S. Patent No. 4,737,579 relates to monoclonal antibodies which define antigens associated with human non-small cell lung carcinomas, to diagnostic and therapeutic methods.

U. S. Patent No. 4,748,112 relates to regression associated antigens identified from neoplastic cells, their use in monitoring cancer patients, treatments and the production of antibodies.

U. S. Patent No. 4,762,800 relates to antibody-producing hybridoma cell lines, monoclonal antibodies from these cell lines and their use in detecting and differentiating between normal and cancerous cells.

## BACKGROUND OF THE INVENTION

The cytochemical diagnostic test commonly known as the "PAP Smear" is widely recognized as one of the most effective preventions for invasive cancer. The PAP test is based on the identification and characterization of "epithelial" cells obtained by gently brushing or scraping the uterine lining in the region of the cervix.

The epithelial cells constituting the cervix/uterine lining represent the potential progenitors of benign warts and polyps as well as life threatening invasive cancers.

Normal epithelial cells have a uniform, polygonal shape. The nucleus is essentially circular and relatively small compared to the whole cell. The nuclear contents are evenly distributed and manifest a very fine, granular appearance.

Variation in these features provide the basis for identification of epithelial cells derived from lesions which might become cancer (dysplasia), lesions which will inevitably become cancer (CIN: cervical intraepithelial neoplasia), and lesions which have already progressed to invasive cancer.

At present, cervical cancer screening programs (e.g. cytologic evaluation of exfoliated cells and histological examination of biopsy or surgical specimens) can provide accurate diagnosis for premalignant and malignant lesions. It is widely known that only some dysplastic epithelial changes of lower grade, are destined to undergo a malignant transformation. Cytology and. histology, which rely mainly on a morphologic evaluation of atypia cannot reliably predict the rate of progression of a known dysplastic lesion.

Furthermore, it is reasonable to assume that the evolutive precancerous lesions and those that can regress may be somehow distinguishable. Therefore, the identification of a marker capable of selecting patients at high risk for malignant cancer would be of utmost clinical significance in advancing our understanding of the histologic events that regulate cervical tumor progression and in guiding the choice and management of a proper therapeutic approach.

Recent studies of viral etiology of cervical cancer seem to be promising in defining new antigens that are specifically related to evolutive cervical lesions. In this context, an increasing number of multidisciplinary studies provides evidence for HSV-2 as an oncogenic virus that plays a significant role in cervical carcinogenesis [Aurelian et al (1981) Cancer 48:455-471; Aurelian (1983) in Gynecologic Oncology. C.T. Griffiths and A.F. Fuller eds.; Aurelian (1986) in Herpes and Papillona Viruses. G. dePalo, F. Rilke and H. zur Hausen eds.].

Inactivated HSV-2 or its DNA can induce CIN and invasive cancer in cervically infected mice [Wentz, W.B. et al 1983: Science 222:1128-1129]. The viral transforming sequences have been identified and code for a protein, designated AG-4, identical to ICP-10 the large (144,000 dalton) component of viral ribonucleotide reductase that is expressed in transformed cells. [Jariwalla et al (1980) PNAS 77:2279-2283; Manak, M. et al (1981) J. Virol 40:289-300; Hayashi, Y. et al (1985) PNAS 82:8493-8497; Jones. C. et al (1986) PNAS 83:7855-7859].

Antibodies to AG-4 have been detected in sera of cervical cancer patients and in cervical tumor tissues in indirect immunofluorescence and in immunoperoxidase assay using monoclonal antibodies that specifically recognize ICP-10 [Aurelian et al (1983) 1:301-313; Costa et al (1987) Cancer Detection and Prevention 1:189-205].

Further evidence for the association of HSV-2 with cervical cancer is the finding that 10-30% of these carcinomas contain specific fragment of HSV-2 DNA not detectable in healthy cervical tissues [Kitchener, H.C. (1988) Brit J. Obstet Gynec 95:182].

More recent transformation studies (Aurelian, unpublished) have identified the segment of the HSV-2 genome encompassing the ICP-10 promoter and sequences encoding the amino terminal portion of the ICP-10 protein as the region functionally relevant to the transforming potential of HSV-2.

Within this region, the segment between amino acids 13 and 26 was predicted to possess great immunological potential. A homologous peptide (LA-1) was synthesized and used to prepare monospecific antiserum.

In addition to HSV-2, cellular oncogenes (c-onc) or protooncogenes, counterparts of transforming retroviral oncogenes, have attracted considerable attention and have been proposed as significant determinants of tumor progression. The cellular myc (c-myc ) protooncogene has been found to be amplified in a large number of human cancer cell lines [Slamon, J. D. (1984) Science 224:256-262].

In a recent study, Ocadiz et al [(1987) Cancer Res. 47:4173-4177] detected c-myc amplification and/or rearrangement in 90% of cervical cancers, but not in normal tissues, suggesting a possible involvement of c-myc in at least some steps of cervical oncogenesis.

Other studies have focused in the cell growth promoting activity of the cellular phosphoprotein, p53. It was reported that p53 protein level are elevated in many human and mouse transformed cell lines, including those transformed by viruses, thus implying that p53 gene may have an oncogenic potential [Pohl, J. et al (1988) Mol Cell Biol 8:2078-2081].

The role of Epidermal Growth Factor (EGF) in inducing proliferation and differentiation of normal epithelial and mesenchymal cell types has been extensively studied and established [Stoscheck, C.M. and King, L.E. (1986) Cancer Res. 46:1030-1037]. EGF exerts its effects through interaction with a receptor (EGF-r) identified as a glycoprotein located at the surface of target cells and coded by the protooncogene c-erts B.

Although EGF has been observed to enhance chemical transformation in vivo and viral transformation in vitro , studies concerning EGF-r detection in cancer cells have shown that its level may be increased, decreased or not changed as compared to normal cells, thereby raising questions as to its involvement in carcinogenesis and its value as a marker.

It is clear that reliable diagnostic-prognostic markers for the management of cervical intraepithelial neoplasia are still required.

## OBJECTS OF THE INVENTION

One of the objects of this invention is to provide a material called LA-1 and composed of amino acids as follows: N-Ala-Arg-Ser-Pro-Ser-Glu-Arg-Gln-Glu-Pro-Arg-Glu-Pro-Glu-Cys--C. It has antigenic activity which is associated with human intraepithelial neoplasia.

Another object of this invention is to provide a system which includes the peptide LA-1, or a related peptide, and an antibody to the peptide.

Another object of this invention is to provide a system which includes antigenic peptides, which are functionally related to the LA-1 peptide and which are encoded in the 2.1 Sac I/Stu I kilobase fragment of the HSV-2 genome (map units 0.554-0.570) that has neoplastic potential, and an antibody to such peptides.

And another object of this invention relates to a system which as diagnostic-prognostic potential for identifying the carcinoma in situ patient at high risk of developing invasive cervical cancer.

Still another object of this invention relates to the manufacture of antibodies produced in response to the peptide LA-1 and the related peptides.

In another aspect, this invention relates to the manufacture of antibodies produced in response to variations of the peptide LA-1 and the related peptides.

In an additional significant aspect, the invention relates to use of the antibodies to identify antigens.

An additional aspect of the invention is the production of antibodies using such antigens.

In an additional significant aspect, the invention relates to use of the antibodies to identify antigens. An additional object of the invention is production of antibodies using such antigens.

A further object of this invention is to provide antibodies labeled with a cytotoxic substance.

Yet another aspect of this invention relates to antibodies labeled with a substance capable of producing a detectable signal.

And another object of this invention relates to antibodies attached to therapeutic agents.

This invention relates to methods of identifying cancerous cells from human cervical intraepithelial neoplasia.

According to this invention, practical processes have been discovered for diagnosing human cervical intraepithelial neoplasia.

Another significant aspect of this invention relates to methods of identifying precancerous cells from human subjects.

## DESCRIPTION OF THE FIGURES

These and other objects of the invention will become more apparent and obvious from the accompanying selected experiments and figures (photos) wherein:

Fig. 1 is a photograph of amino acid sequence of the synthetic peptide LA-1;

Fig. 2 is a photograph of immunoprecipitation of extracts from HSV-2 infected MRC-5 cells labeled with 35-S-methionine;

Fig. 3 is a photograph of a western blot of HeLa and Caski cells;

Fig. 4 is a photograph of a western blot of artificial mixtures of mock infected and HSV-s infected HeLa cells;

Fig. 5 is a photograph of ICP-10 and 60kDa protein from HeLa cells;

Fig. 6 is a photograph of a western blot of proteins from invasive cancer and normal cervical tissue.

## A DETAILD DESCRIPTION OF THE FIGURES

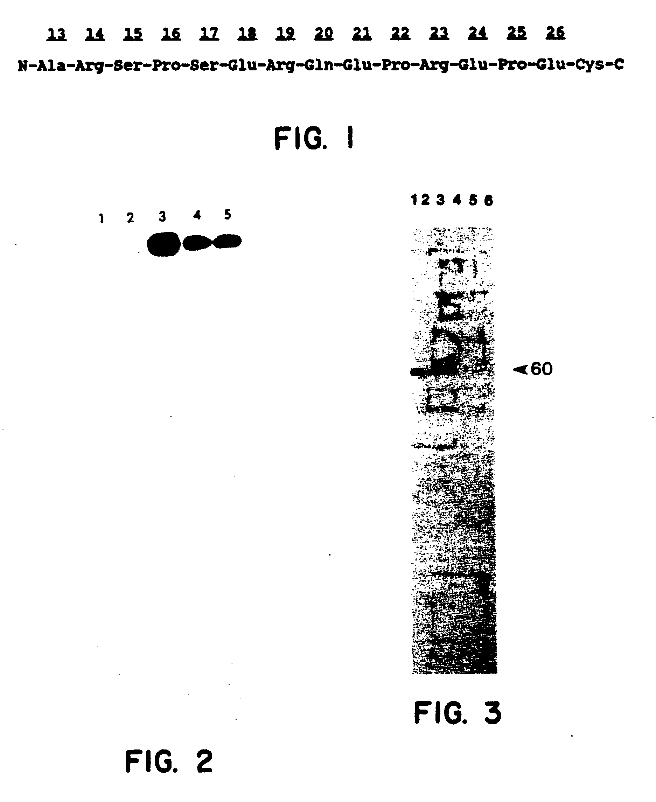

Fig. 1 shows the amino acid sequence of the synthetic peptide LA-1. Cysteine was added to the carboxy terminus to facilitate coupling to a protein carrier. The numbers refer to the residue numbers of the mature 144kDa (ICP-10) protein;

Fig. 2 shows immunoprecipitation of extracts from HSV-2 infected MRC-5 cells labeled with 35S-methionine (3-8 hrs. p.i.). Preimmune serum (Lane 1), anti-LA-1 serum absorbed with ICP-10 (Lane 2), anti-LA-1 serum (Lane 3), MAb 30 (Lane 4), MAb 48S (Lane 5);

Fig. 3 is a western blot of HeLa (Lanes 1, 2) and Caski (Lanes 3, 4) cells (200$\mu$g protein/well) assayed with anti-LA-1 (Lanes 1, 3) or preimmune (Lanes 2, 4) serum (1:10 dilution) and MRC-5 cells at 200$\mu$g protein/well (Lane 5) and 50$\mu$g protein/well (Lane 6) assayed with anti-LA-1 serum;

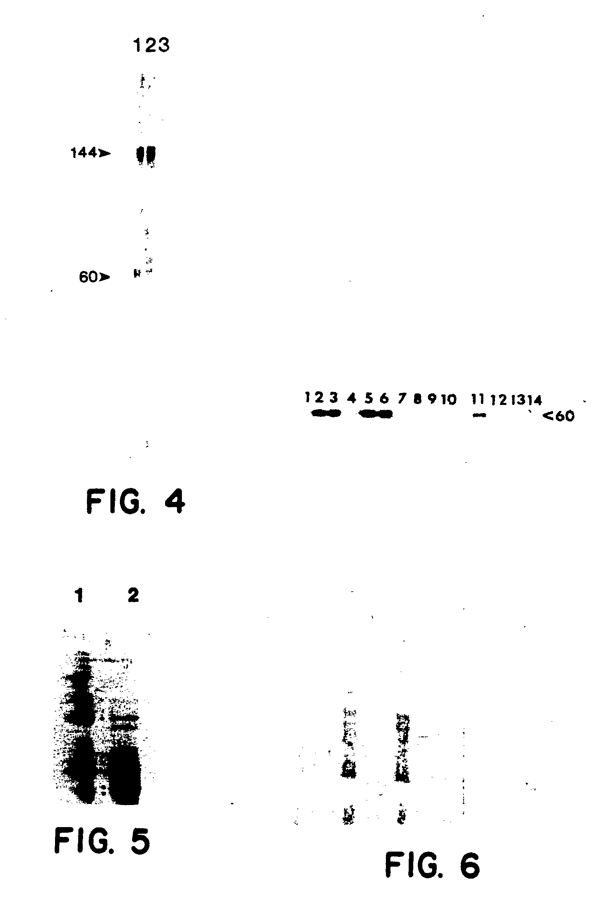

Fig. 4 is a western blot of artificial mixtures of mock infected and HSV-2 infected Hela cells (100$\mu$g protein/well) reacted with anti-LA-1 serum (Lanes 1, 2) or anti-LA-1 serum absorbed with ICP-10 (Lane 3);

Fig. 5 is a tryptic fingerprint analysis of ICP-10 (Lane 1) and the 60kDa protein from HeLa cells (Lane 2);

Fig. 6 is a western blot of proteins from invasive cancers (Lanes 2, 3, 5, 6, 11), CIN III (Lanes 8, 9) and normal cervical tissue (Lanes 13, 14) with anti-LA-1 serum (1:10 dilution). Preimmune serum (Lanes 7, 10) and serum absorbed with the HeLa 60kKa protein (Lanes 1, 4) did not react.]

## DESCRIPTION OF THE EXPERIMENTS

### 1. Materials and Methods

Cells: Human embryo lung (MRC-5), HeLa and Caski cells were grown in Dulbecco's Modified Minimum Essential Medium (Gibco Laboratories, Grand Island, NY) with 10% fetal calf serum (FCS) (Armour Pharmaceutical, Kankakee, Ill.).

Antibodies: MAbs 48S and 6A6, specific for the HSV-2 ICP-10 protein were obtained from Dr. Zweig (NCI, Frederick, MD.) and Syva Micro (Palo Alto, CA) respectively. MAb PAb421, specific for p53, was the gift of Dr. Harlow (Cold Spring Harbor Labs, NY). MAb EGFr, specific for the epidermal growth factor receptor, was purchased from ICN Biomedicals (Lisle, Ill.). MAb Mycl-6E10, specific for c-myc was the gift of Dr. G. Lewis (University of Maryland). Antibody to HPV-16 E6 protein was the gift of Sclavo (Siena, Italy). The preparation of MAb 30 is described separately.

Peptide synthesis and antiserum preparation: Peptide LA-1, corresponding to residues 13-26 of the HSV-2 ICP-10 protein (fig. 1) was synthesized using Merrifield solid phase methods (Stewart and Young, 1969: Solid Phase Peptide Synthesis. W. H. Freeman and Co., San Francisco). Antibody was prepared in rabbits using KLH conjugated LA-1 [Liu et al , (1979) Biochem 18:690].

Protein extraction: Minced tissues were suspended in 10mM Tris-HCl (pH 7.5) with 1% Triton X-100, 0.01% sodium deoxycholate, 0.1M NaCl, 5mM EDTA and 1mM phenyl methyl sulfonyl fluoride (PMSF) at a concentration of 200 mg/0.3ml. Tissue culture cells ($2 \times 10^6$/ml) were resuspended in lysis buffer [10mM Tris-HCl (pH 8.0), 0.15M NaCl, 1% Nonidet-40 (NP-40), 0.1% sodium dodecyl sulfate (SDS), 1% sodium deoxycholate, 1mM dithiothreitol (DTT) and 1mM PMSF]. Cells were disrupted by hemogenization and sonication. The supernatants were clarified by centrifugation (30,000 x g; 30 min).

Western blot assay: Extracts were boiled (5 min) in 1/3 volume of denaturing solution [150mM Tris-HCl, pH 7.0, 5.7% SDS, 14% 2-mercaptoethanol (2-ME), 17% sucrose, 0.04% bromothymol blue]. Proteins were resolved on 8.5% SDS-polyacrylamide gels (SDS-PAGE) and electroblotted onto BA85, 0.45 micron nitrocellulose transfer membranes (Schleicher and Schuell, Keene, NH) in 25mM Tris-HCl, 192mM glycine and 20% (v/v) methanol at 100 V for 4 hrs. The blots were pre-soaked (60 min, 37 C) in TN buffer (10mM Tris-HCl, pH 7.4, 0.15M NaCl) with 5% bovine serum albumin (TN-BSA) followed by TN-BSA with 0.05% Tween 20 (15 min. 24 C). They were incubated (90 min, 24 C) with anti-LA-1 serum (1:10 to 1:100 in TN-BSA with Tween), washed and incubated (90 min; 24 C) with 2 g/ml Protein-A-Peroxidase (Sigma). The reaction was developed by exposure (20-30 min; 24 C) to a 33 g/ml O-dianisidine solution (Sigma in 0.01%

$H_2O_2$, 10mM Tris-HCl, pH 7.4.

Peptide mapping: Tryptic peptide analysis was performed by the method of Cleveland (1977). Proteins were resolved on 8.5% SDS-PAGE. Bands were excised, soaked (30 min) in 0.125M Tris-HCl (pH 6.8), 0.1% SDS and 1mM EDTA, overlayed with 25 g/ml of Staphylococcus aureus V-8 Protease (Sigma) (prepared in the same buffer with 15% glycerol) and electrophoresed on 12.5% gels. For fluorography gels were treated with Enhance (Dupont) and exposed to Kodak X-Omat AR film at -80 C.]

Immunohistochemistry: Formalin fixed tissue sections were deparaffinized and stained as described (Costa et al , 1987: Cancer Detection and Prevention 1:189). They were treated with 100% methanol/3% $H_2O_2$ (40 min) and normal horse serum (15 min) and exposed (60 min 24 C) to primary antibodies. Anti-HPV-16 E6 was also assayed for 15 hrs. at 24 or 37 C. Biotinilated secondary antibody (horse anti-IgG) and Avidin-Biotin peroxidase complex were obtained from Vector Labs, Burlinham CA. The peroxidase reaction was developed (5 min) in fresh 0.02% 3,3'-diamino-benzidine tetrahydrochloride with 0.001% $H_2O_2$.

Study groups: Serial sections (4) of biopsies used in immunohistochemistry were obtained from 62 patients with CIN or invasive cervical cancer, 7 women with normal cytology (cervicitis and/or metaplasia) and 33 patients with other squamous cancers (volva, lung, penis, pharynx, larynx, vocal cord, tongue, floor of mouth, skin, oesophagus). Tissues for Western blot were obtained from 6 of the cervical cancer and 2 of the CIN III patients at surgery, and from 2 normal women at hysterectomy for uterine fibroids.

## 2. Results

Specificity of anti-LA-1 serum: If HSV-2 related functions fulfill the criteria of a marker for CIN progression, they probably reside within the amino-terminal sequences of ICP10 encoded by transforming HSV-2 DNA sequence [Hayashi et al , (1985) Proc. Nat. Acad. Sci. 82:8493]. Secondary structure predictions based on the algorithm of Chou and Fasman [(1978) Adv. ENZ 47:45], identified the LA-1 sequence (Fig. 1) as a highly hydrophilic region possessing strong alpha-a-helical and beta-B-turn characteristics. Based on the assumption that such regions possess immunological potential [Hopp and Woods, 1981: PNAS 78:3824], this sequence was selected for peptide synthesis. As shown in Fig. 2, a 144kDa protein [consistent with ICP10 (Hayashi et al , 1985)] was precipitated by anti-LA-1 serum from extracts of HSV-2 infected MRC-5 cells labeled with 35A-methionine at 3-8 hrs. p.i. (Lane 3). The protein was also precipitated by MAbs 30 and 48S (Lanes 4,5).

LA-1 determinant is localized on 60kDa protein: Increasing concentrations (50-200 g protein/well) of Hela and Caski cell extracts (adenosquamous and squamous cervical cancer respectively) and normal MRC-5 cells were assayed with anti-LA-1 serum by Western blotting (Fig. 3). One weakly reactive 60kDa band was seen in MRC-5 cells at 200 (Lane 5) but not 50 (Lane 6)μg protein/well. A much stronger 60kDa band was seen in HeLa and Caski cells (Lanes 1,3) at all concentrations, suggesting that the 60kDa protein is over-expressed in cancer as compared to normal cells. Significantly, adsorption of the anti-LA-1 serum with nitrocellulose blots containing ICP10 (6 cycles, 1 hr; 37 C) abrogated its reactivity for both the 144kDa and 60kDa proteins (Fig. 4, Lane 3). Tryptic peptide analysis (Fig. 5) indicated that the two proteins are structurally distinct, which is not surprising given their different molecular weights. However 6 of the 9 peptides in the 60kDa protein were similar to ICP10 peptides both in terms of their relative intensity and migration.

Related antigenic peptides encoded in HSV-2 genome: Further studies have identified functionally related antigenic peptides encoded in the 2.1 kilobase region of the HSV-2 genome that has neoplastic transformation potential. This region, which encodes the LA-1 peptide, is defined as the Sac I/Stu I restriction endonuclease fragment and spans units 0.554 through 0.570 on the HSV-2 genome map.

Reactivity of anti-LA-1 serum with clinical tissues: Staining with anti-LA-1 serum was observed in 7 of 15 (47%) cases of CIN I, 13 of 29 (45%) CIN II, 6 of 12 (50%) CIN III and 4 of 6 (67%) invasive cancers. This compares to 1/7 (14%) normal tissues without atypical features and 1/33 (3%) squamous cancers at other body sites (Table 1). Preimmune serum and MAb 6A6 were negative. However virtually identical staining patterns were obtained with MAbs 30 and 48S. Staining was primarily cytoplasmic. It was specific for the atypical epithelium and was completely absent from the surrounding tissue. The intensity and distribution of the staining did not correlate with the severity of the lesion. However intranuclear antigen localization was primarily detected in CIN III and invasive cancers.

Tissues from 6 of the LA-1 positive invasive cancer patients, 2 CIN III patients and 2 normal women were also assayed with anti-LA-1 serum in Western blot. The 60kDa band was observed in all cancers (Fig. 6, Lanes 2, 3, 5, 6, 11) and in one CIN III (Lane 8). It was not seen in normal tissues (Lanes 13, 14) nor in cancers assayed with serum absorbed with the HeLa cell 60kDa protein (Lanes 1, 4).

Staining with anti-HPV-16 E6 and MAbs to protooncogenes: Serial sections of the tissues stained with anti-LA-1 were studied. HPV-16 E6 antibody failed to stain all the tissues in these series, under all experimental conditions. EGF-r was expressed in all tissues, but expression was reduced in CIN (20-33%) and cervical cancer (50%) as compared to normal tissues and other squamous cancers (86 and 75% respectively). Reactivity, localized on the cell membrane, was primarily in the epithelium but it was also in glandular and vascular endothelium. p53 was expressed in a low proportion (14-17%) of tissues independent of pathology, while c-myc was expressed only in CIN III and invasive cancers but at low (13-14%) frequency. It was localized in atypical nuclei (Table 1).

Table 1

| Expression of La-1 and Protooncogenes in Clinical Tissues. | | | | |
|---|---|---|---|---|
| Diagnosis | Percent cases staining with | | | |
| | LA-1 | EGFr | p53 | C-myc |
| CIN I/II | 46 | 33 | 15 | 0 |
| CIN IJI | 50 | 20 | 17 | 13 |
| Invasive cervix cancer | 67 | 50 | 0 | 14 |
| Normal cervix | 14 | 86 | 17 | 0 |
| Other squamous cancers | 3 | 75 | 25 | ND |

## 3. Discussion

The LA-1 determinant corresponding to 13 residues on the HSV-2 ICP10 protein is also located on a 60kDa cellular protein that is overexpressed in cervical cancer and its precursor lesions, as determined by Western blotting and immunoperoxidase staining.

The 60kDa protein is distinct from ICP10. However the degree of similarity is rather extensive as evidenced by peptide mapping and the similar staining patterns obtained with MAbs 30 and 48S specific for ICP10 antigenic determinants distinct from LA-1.

Intranuclear localization may be the significant determinant of progression. This is particularly attractive under the hypothesis that the 60kDa protein, with the LA-1 determinant, is the host cell factor which facilitates neoplastic growth.

The frequency of LA-1 positivity correlates with the histologic degree of severity of the neoplastic lesions. Expression of HPV-16 E6 or the protooncogenes c-myc , EGF-r or p53 does not correlate with the histologic degree of severity or CIN progression. c-myc was expressed only in advanced lesions.

EGFr expression was decreased in cervical lesions as compared to normal cervic and other cancers. Expression of p53 was uniformly low. Anti-E6 serum did not stain under all assay conditions. Since it was prepared with bacterially derived fusion protein and it precipitates E6 from tissue culture cells, the negative findings presumably reflect low levels (or absence) of E6 expression in clinical tissues.

In separate experiments (te Velde and Aurelian, 1987: Tumor Biol 8: 26-33), we have evaluated the potential clinical value of AG-4 (equivalent of the 144 kDa, ICP-10 protein) antibodies in patients with established cervical cancer in terms of (a) the assessment of prognosis before treatment and (b) monitoring the course of the cancer process during long-term follow-up. Forty-seven patients were investigated during a median follow-up time of 5.4 years. During this time, 25 developed recurrent disease. The presence of AG-4 antibodies during follow-up was associated with recurrent disease (p 0.001). In most cases, the presence of AG-4 antibodies preceded the clinical diagnosis of recurrence.

These experiments indicate that immunohistochemistry with anti-LA-1 serum may prove to be a highly sensitive method for identification of a precursor lesion with malignant potential.

It is intended that the scope of the claims appended hereto be construed as encompassing all the features of patentable novelty which reside in the present invention, including variations which would be treated as equivalents thereof by those skilled in the art to which this invention pertains.

The features disclosed in the foregoing description, in the claims and/or in the accompanying drawings may, both, separately and in any combination thereof, be material for realising the invention in diverse forms thereof.

**Claims**

1. As an article of manufacture, a synthetic peptide comprised as follows: N-Ala-Arg-Ser-Pro-Ser-Glu-Arg-Gln-Glu-Pro-Arg-Glu-Pro-Glu-Cys-C.

2. As an article of manufacture comprising antibodies produced in response to the peptide: N-Ala-Arg-Ser-Pro-Ser-Glu-Arg-Gln-Glu-Pro-Arg-Glu-Pro-Glu-Cys-C.

3. As an article of manufacture, antibodies produced in response to a fragment of the peptide: N-Ala-Arg-Ser-Pro-Ser-Glu-Arg-Gln-Glu-Pro-Arg-Glu-Pro-Glu-Cys-C.

4. As an article of manufacture, antibodies produced in response to a peptide which mimics the peptide: N-Ala-Arg-Ser-Pro-Ser-Glu-Arg-Gln-Glu-Pro-Arg-Glu-Pro-Glu-Cys-C.

5. As an article of manufacture, antibodies produced in response to a peptide which mimics a fragment of the peptide: N-Ala-Arg-Ser-Pro-Ser-Glu-Arg-Gln-Glu-Pro-Arg-Glu-Pro-Glu-Cys-C.

6. As an article of manufacture, antibodies produced in response to peptides encoded by the 2.1 kilobase region of the HSV-2 genome which also encodes the peptide: N-Ala-Arg-Ser-Pro-Ser-Glu-Arg-Gln-Glu-Pro-Arg-Glu-Pro-Glu-Cys-C.

7. As an article of manufacture, antibodies produced in response to proteins identified through use of antibodies recited in claim 2.

8. As an article of manufacure, antibodies as recited in claim 2, attached to a moiety of moieties capable of providing a detectable signal or signals.

9. As an article of manufacture, antibodies as recited in claim 2, attached to one or more cytotoxic agents.

10. As an article of manufacture, antibodies as recited in claim 2, attached to one or more therapeutic agents.

11. A method for evaluating the presence of cervical intraepithelial neoplasia in a human or animal host, said method comprising bringing into close association a protein and a probe recognizing said protein, said probe being an antibody produced in response to the peptide: N-Ala-Arg-Ser-Pro-Ser-Glu-Arg-Gln-Glu-Pro-Arg-Glu-Pro-Glu-Cys-C.

12. A method according to claim 11 wherein said probe is antibody as recited in claim 3.

13. A method for evaluating probability of development of cervical intraepithelial neoplasia in a human or animal host, said method comprising bringing into close association a protein and a probe recognizing said protein, said probe being an antibody produced in response to the peptide: N-Ala-Arg-Ser-Pro-Ser-Glu-Arg-Gln-Glu-Pro-Arg-Glu-Pro-Glu-Cys-C.

14. A method according to claim 13 wherein said probe is antibody as recited in claim 3.

15. A method according to claim 11, wherein proteins recognized by the antibody are located in human cells.

16. A method according to claim 11, wherein proteins recognized by the antibody are produced by cultured cells.

<u>13</u>  <u>14</u>  <u>15</u>  <u>16</u>  <u>17</u>  <u>18</u>  <u>19</u>  <u>20</u>  <u>21</u>  <u>22</u>  <u>23</u>  <u>24</u>  <u>25</u>  <u>26</u>

N-Ala-Arg-Ser-Pro-Ser-Glu-Arg-Gln-Glu-Pro-Arg-Glu-Pro-Glu-Cys-C

## FIG. 1

FIG. 2

◄ 60

FIG. 3

FIG. 4

FIG. 5

FIG. 6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | CANCER CELLS 7 / MOLECULAR DIAGNOSITCS OF HUMAN CANCER, 1989, pages 187-191, Cold Spring Harbor Laboratory; L. AURELIAN et al.: "Amino-terminal epitope of herpes simplex virus type 2 ICP10 protein as a molecular diagnostic marker for cervical intraepithelial neoplasia" * Whole article * | 1-16 | C 07 K 7/08 G 01 N 33/574 |
| P,A | JOURNAL OF VIROLOGY, vol. 63, no. 8, August 1989, pages 3389-3398, American Society for Microbiology; T.D. CHUNG et al.: "Protein kinase activity associated with the large subunit of herpes simplex virus type 2 ribonucleotide reductase (ICP10)" * Whole article * | 1,10 | |

TECHNICAL FIELDS
SEARCHED (Int. Cl.5)

C 07 K
G 01 N

The present search report has been drawn up for all claims

| Place of search | Date of completion of the search | Examiner |
|---|---|---|
| THE HAGUE | 16-08-1990 | RAJIC M. |

EPO FORM 1503 03.82 (P0401)